# EUROPEAN PATENT APPLICATION

(11) **EP 0 705 843 A1**
(43) Date of publication of application: **10.04.1996**
(21) Application number: 94924890.0
(22) Date of filing: 29.08.1994
(51) Int. Cl.: C07K 14/315, A23L 3/3526

(54) **BACTERIOCIN FROM ENTEROCOCCUS FEACIUM ACTIVE AGAINST LISTERIA MONOCYTOGENES**

(30) Priority: 31.08.1993 ES 9301882
(71) Applicant: INSTITUT DE RECERCA I TECNOLOGIA AGROALIMENTARIES (IRTA), E-08008 Barcelona (ES); Casa Tarradellas, S.A., E-08500 Vic (ES)
(72) Inventor: HUGAS MAURICI, Marta, E-08500 Vic (ES); GARRIGA TURON, Margarita, E-08500 Vic (ES); MONFORT BOLIVAR, Josep M, E-08500 Vic (ES); YLLA ULLASTRE, Josep, E-08500 Vic (ES)
(74) Representative: Urizar Anasagasti, José Antonio
(86) International application number: ES9400081
(87) International publication number: WO9506663

(57) **Abstract**

The present invention relates to a new bacteriocine against *Listeria monocytogenes*, obtained from the culture of a new strain of *Enterococcus faecium* named CTC492. The bacteriocine is a peptide having a molecular weight of 4,282 dalton and comprises a sequence of 35 aminoacids and may be applied with substantial efficiency for preventing and avoiding the growth and propagation of *Listeria monocytogenes* in food, particularly in meat and meat products and in milk and milk products.

## Description

### FIELD OF THE TECHNIQUE

The present invention refers to a new bacteriocin, obtained from a new strain of Enterococcus faecium, with a bactericide action against Listeria monocytogenes and with an application for inhibiting growth and propagation of said patogenic microorganism on foods, especially on meat and meat products and on milk and diary products.

### PRIOR ART

Listeria monocytogenes is a patogenic microorganism that produces severe upsets on human beings and animals and can be easily transmitted by means of contaminated foods, especially by means of meat and meat products and milk and dairy products. Said patogenic microorganism shows to be cooling resistant but results sensitive to acid conditions, so that low pH values.

In many cases, it is not possible to store and offer foods with a low pH value. In this way, nowadays, the consumer refuses those meat products which are too acid and his preferences are directed to fermented meat products with a low acid taste, then resulting in a sizeable increase of possible contamination and growth of Listeria monocytogenes on them.

In accordance with Tagg, J.R. et al.; Bacteriol. Review, 40; 722-756 (1976), the term "Bacteriocin" refers to peptide-like substances, produced by microorganisms, able to specifically inhibit the growth of other bacteria by means of absortion to their specific receptors.

There have been various active bacteriocins disclosed against Listeria monocytogenes, useful for combating food contamination by the same. In this way, Vendenbergh et al. (European Patent Number 0 326 062) describe one method for inhibiting Listeria monocytogenes by using a bacteriocin obtained from a strain of Pediococcus acidilactici. The same authors disclose in the European Patent Application Number 0 453 719 that the bactericide activity is related to a 4,629 molecular weight fragment, isolated from the original 16,500 molecular weight bacteriocin.

Marugg et al. describe in the European Patent Application Number 0 493 779 a cloned gene encoding for a bacteriocin in Pediococcus acidilactici, useful for inhibiting the growth of Listeria on foods. Kawase, Kozo et al. describe in the European Patent Application Number 0 503 939 an antimicrobian peptide capable of inhibiting Listeria monocytogenes, among other microorganisms, obtained from bovine Lactoferrine hydrolysis.

On the other hand, it is known that bacteria of Streptococcus faecium species, nowadays called Enterococcus faecium, are capable of producing low molecular weight substances with antimicrobian activity, which are not bacteriocins and are useful in preventive and curative kind therapeutical treatements. In this way, Kawai, Yasuo et al. disclose, in Published Japanese Patent Applications Numbers: 6153293 and 61109728, the use of a compound, having a molecular formula of C₁₁H₁₅O₅N₅, obtained from Streptococcus faecium, in toothcare products. In turn, Lewenstein et al. disclose, in European Patent Application Number 0 405 569, a product having antimicrobian activity, obtained from Streptococcus faecium cultures, for the treatement and prevention of the gastrointestinal disorders caused by, among other microorganisms, bacteria of genus Listeria. Said product has a molecular weight lower than 1,000 and it is not a bacteriocin.

Bacteriocins against Listeria monocytogenes described heretofore have various advantages and drawbacks but there still remains the necessity to find alternative high efficiency and availability ways which would allow the Food Industry to efficiently attack the risks of contamination of its products by Listeria monocytogenes.

The authors of the present invention have discovered a new bacteriocin produced by a new strain of Enterococcus faecium, with a surprising specific activity against Listeria monocytogenes, and which is very accessible and easy to be obtained.

### OBJECT OF THE INVENTION

The present invention is directed to provide a new bacteriocin against Listeria monocytogenes, highly effective combating the growth and propagation of Listeria monocytogenes on foods.

Another object of the present invention consist of providing a new bacterial strain of Enterococcus faecium capable of producing said bacteriocin.

A further object of the present invention comprises the use of said bacteriocin or said bacterial strain in order to prevent and avoid the growth and propagation of Listeria monocytogenes in foodstuff.

### DESCRIPTION OF THE INVENTION

The bacteriocin against Listeria monocytogenes which is object of the present invention is characterized by being a peptide with a molecular weight larger than 4.000, obtained by means of culturing the bacterial strain CTC492 of Enterococcus faecium. CTC492 is the designation provided to said strain by the authors of the present invention and it corresponds to the *"Colección de Cultivos del Centre de Tecnologia de la Carn, Institut de Recerca i Tecnologia Agroalimentáries"* (Culture Collection of The Centre of Meat Technology, Food-Farming Research & Technology Institute), domiciled in Granja Camps i Armet s/n, 17121 Monells (Gerona), Spain.

A culture of said strain has been deposited in accordance with Budapest Treaty in relation to international acceptance of microorganism deposits towards the procedure on the subject of the CECT, *Colección Española de Cultivos Tipo, de la Universidad de Valencia* (Spanish Culture Colection, University of Valencia), domiciled in Campus de Burjasot, 46100 Burjasot (Valencia), Spain, which has assigned the deposit-number CECT 4453 to the same.

The strain CTC492 of Enterococcus faecium comprises Gram-positive, Catalase-negative non sporulated cocci whose principal fermentation product is Lactic acid and which are capable of producing α-hemolysis in blood-agar (48 hours at 30ºC). This strain ferments Melibiose; it is unable to ferment Melezitose and does not reduce Tripheniltetrazolium (TTZ) in a 0,1% Thallium acetate medium. It presents a positive growth at 45ºC and in 6,5% Sodium Chloride and positively reacts with D-antigen. Its profile of several sugars fermentation is as follows:

| | | |
|---|---|---|
| Glucose + | Ribose + | Melibiose + |
| Mannose + | Celobiose + | Ramnose - |
| Saccharose + | D-Rafinose - | Melezitose - |
| Lactose + | Maltose + | Trehalose + |

All these characteristics agree with those described in "Bergey's Manual of Systematic Bacteriology", vol 2, Baltimore; William and Willains (1986) for Enterococcus faecium, formerly Streptococcus faecium, clasification.

Members of Enterococcus faecium group are passed by The International Diary Products Federation as Food quality bacteria useful as starter cultures for dairy products.

Strain CTC492 can be cultured with an excellent yield on MRS culture broth (lactobacilli culture broth according to De Man, Rogosa and Sharpe commercialized by companies DIFCO, OXOID, MERCK, etc.) in aerobic conditions at a temperature of about 30ºC.

The bacteriocin against Listeria monocytogenes which is object of the present invention, obtained from strain CTC492, has a molecular weight of 4,282 Dalton and is a peptide that comprises 35 aminoacids in the following sequence:

Thr-Thr-His-Ser-Gly-Lys-Tyr-Tyr-Gly-Asn-Gly-Val-
Tyr-Cys-Thr-Lys-Asn-Lys-Cys-Thr-Val-Asp-Thr-Ala-Lys-Ala-
Thr-Thr-Xaa-Ile-Ala-Gly-Met-Ser-Ile.

Said amino-acid sequence is new and presents certain resemblance with the bacteriocins Pediocin, Sakacin-A, Leucocin-A and Curvacin-A.

By using a degenerated probe which comprises residues from 6 to 15, the authors of the present invention have been able to determine, by said probe hybridation with the chromosomic and pasmidic DNA of strain CTC492, that the producing gene of the bacteriocin object of the present invention may be located in a chromosomic fragment EcoR1 of 12 to 14 Kb.

The bacteriocin object of the present invention, designated as Entericin CTC492 by its authors, becomes inactive by means of treatment with proteolytic enzimes such as Tripsine, Nagarse or Proteinase K, but it is resistant to treatement at temperature of 100ºC for 20 minutes.

Whole strain CTC492 as much as the bacteriocin (Entericin CTC492) culture are capable of inhibiting the growth of Listeria monocytogenes, Listeria innocua and Listeria welshimeri in agar drop assay, agar difussion assay and direct antagonism assay under conditions in which the inhibition is suppressed due to Hydrogen Peroxyde and acid. Inhibitory action is of bactericide-kind.

Assays of growth inhibition have also been carried out for various strains of Listeria in contaminated food products, e.g., contaminated crushed-meat doughs, with a drastic decrease in number of colony forming units (cfu) observed, in periods of about 24 hours, after the addition of Entericin CTC492 of the invention.

Entericin CTC492 may be obtained by the procedure consisting of cultivating the strain CTC492 of Enterococcus faecium on MRS broth, culture centrifugation to obtain the supernatant, cold treatement of the same with Ammonium sulphate and subsequent centrifugation of the same to recover the sediment which consists of a highly active concentrate of Entericin CTC492, that can be re-suspended in a suitable amount of Phosphate buffer, pH 7,0.

The activity against Listeria is measured in Activity Units, AU, in accordance with the method described by Barefoot, S. et al. in Appl. Environ. Microbiol. 45 (6), 1808-1815 (1983), conveniently adapted. Accordingly, an AU is defined as the reciprocal of the highest dilution which shows complete inhibition of the strain of Listeria monocytogenes on a culture of approximately 100,00 cfu/ml. The AU are expressed in millilitre (AU/ml) in the case of liquid preparations or in grammes (AU/g) in the case of solid preparations.

In relation to chemical and biochemical recognition studies, the obtained Entericin CTC492 concentrate can be additionally purified by means of a protocol of four steps comprising its precipitation in Ammonium acetate solutions, Ion Exchange Chromatography, Hydrophobic Interaction Chromatography and Reverse Phase Chromatography in the FPLC system (Fast Performance Liquid Chromatography), eluting in a 30% Isopropanol isocratic gradient in a C2-C18 column.

Purified Entericin CTC492 molecular weight is determined by SDS-PAGE (Sodium Dodecylsulphate-Polyacrilamide Gel Electrophoresis) and by Mass Spectrometry.

Entericin CTC492, object of the present invention, can be obtained by known genetic-engineering techniques too; for example, by cloning an auxiliar strain of any suitable microorganism with a vector plasmid obtained from the chromosomic fragment responsible for production of said Entericin CTC492.

The Entericin CTC492 concentrate obtained by procedures mentioned herein above may be used in foods, as a liquid suspension or in a solid form, by drying and mixing it with any kind of appropriate excipient or by undergoing a lyophilization procedure, using sufficiently known techniques in every case.

Also the culture of the strain of Enterococcus faecium itself in a frozen or not, liquid, dried or lyophilized form, as well as any preparation resulting from the same having different activity levels can be used for the application in foods.

Entericin CTC492 as well as the culture of strain CTC492 of Enterococcus faecium or preparations coming from it can be used with a high effectiveness to, thanks to its addition into foods by any suitable known way, prevent and avoid the growth and propagation of Listeria monocytogenes on them, even when their acididity were low. Due to the innocuousness of Entericin CTC492 and strain CTC492 of Enterococcus faecium, there is no counterindication about the kind of food in which they can be used in priciple, although the use is especially directed to avoid contamination on meat and meat products and on milk and diary products.

Entericin CTC492, the culture of strain CTC492 of Enterococcus faecium or derivable preparations can also be used for the treatement of food containers, thereby forming a protective barrier against contamination by Listeria monocytogenes. For example, they may be incorporated to a food protective film by known techniques, such as those described in European Patent Application Number 0 384 319.

Entericin CTC492 object of the present invention can appear in the form of material compositions which would contain other kinds of natural or non-natural chemicals or biological products which may be appropriate.

### DESCRIPTION OF THE DRAWINGS

The present description is accompanied by a sheet of drawings, to represent, in an explanatory but not restrictive way, the object of the present invention, as follows:

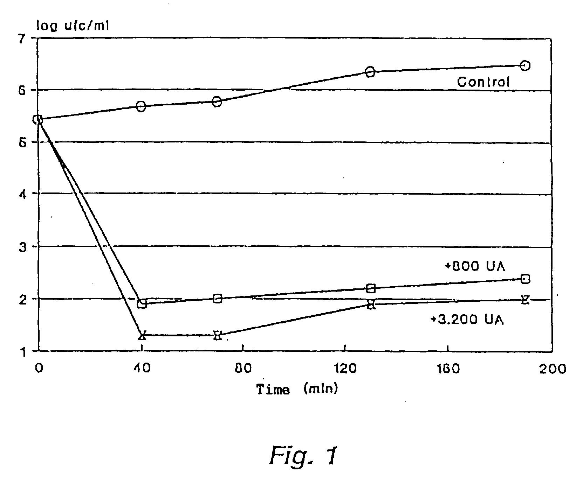

Figure 1 shows a diagram of the effect of the addition of Entericin CTC492 to an exponential phase culture of Listeria monocytogenes. Entericin was added at concentrations of 800 AU/ml and 3,200 AU/ml and the results are shown in a comparative manner with those from a control culture to which no Entericin is added. At the abscissa axis, the lapsed time is measured, whereas at the ordinate axis, the logarithm of the colony forming units, per millilitre (log [cfu/ml]) is shown.

### EXAMPLES:

Following examples are shown also in an explanatory but not restrictive manner for the object of the present invention.

### EXAMPLE 1. OBTENTION OF A ENTERICIN CTC492 CONCENTRATE

500 ml of MRS culture broth of DIFCO Company are inoculated with a 1% of culture volumen in stationary phase of Enterococcus faecium CTC492 on MRS; and resulting culture is then incubated at 30ºC during 12-18 hours. Subsequently, the culture is centrifuged at 10,000 rpm for 10 minutes, the supernatant is collected and a 40% in weight of Ammonium sulfate is added thereto. Resulting mixture is stored between 0ºC and 5ºC for 30 minutes and then centrifuged at 10,000 rpm for 35 minutes. The obtained precipitate is suspended in 10 ml 3mM Potassium Phosphate Buffer at pH 7,0. The obtained suspension constitutes the Entericin CTC492 concentrate which can be used as such or diluted at different concentrations.

### EXAMPLE 2. INHIBITION ASSAY BY AGAR DIFUSSION

An Enterococcus faecium CTC492 culture on MRS broth is centrifuged at 10,000 rpm, incubated for 20-24 hours at 30ºC. The supernatant fluid is collected, its pH is adjusted to 6.5 and it is filter-sterilized through a 0.45 micrometre pore diameter filter.

A 0.2% Glucose MRS soft agar tube is inoculated with 200 microlitres of an overnight culture of Listeria monocytogenes, poured onto a 0.2% Glucose MRS agar plate and allowed to solidify. Holes are then made in the agar by a sterile 3 mm diameter pipette and 30 microlitres of the previously obtained supernatant from the culture of Enterococcus faecium are added into each hole. The plate is incubated in anaerobic conditions at 25ºC for 24 hours and a 10 mm inhibition halo is then observed to be produced arround each hole where the supernatant is deposited.

### EXAMPLE 3. INHIBITION OF LISTERIA MONOCYTOGENES GROWTH BY MEANS OF THE BACTERIOCIN ENTERICIN CTC492.

In the same manner, three exponential phase cultures of Listeria monocytogenes are prepared in a TSBYE medium (triptic soya broth from DIFCO Co.) to which 0.6% yeast extract is added. One culture is reserved as a control and 800 and 3,200 AU/ml Entericin CTC492 prepared as in Example 1 are added to the other two cultures respectively. The cultures are incubated at 30ºC and plate tests are carried out in TSBYE agar at different time intervals, diluting as much as necessary, for counting the Listeria monocytogenes colony former units up. The obtained results, illustrated as log(cfu/ml), are as follows:

| Time (min) | Control | +800AU | +3200AU |
|---|---|---|---|
| 0.0 | 5.43 | 5.43 | 5.43 |
| 40.0 | 5.68 | 1.90 | 1.30 |
| 70.0 | 5.77 | 2.00 | 1.30 |
| 130.0 | 6.34 | 2.20 | 1.90 |
| 190.0 | 6.48 | 2.40 | 2.00 |

In Figure 1, the graphic representation of the obtained values is shown. From its observation, the high efficiency of Entericin CTC492 to inhibit the growth of Listeria monocytogenes is unquestionably deduced.

### EXAMPLE 4. LISTERIA INNOCUA INHIBITION ASSAYS IN MEAT PRODUCTS

Because of security of research workers, these assays were carried out with Listeria innocua, species from genus Listeria that, unlike Listeria monocytogenes, does not produce damages in case of being ingested by humans.

Two shares of meat dough for elaboration of *salchichón* were prepared according to traditional recipe in meat industry, and both were contaminated by Listeria innocua with 10,000 cfu per gramme of meat dough. 800 AU Entericin CTC492, as obtained in Example 1 , per gramme of meat dough were added to one of the shares. Then, putting into skins was carried out.

After 24 hours, cfu/g count decreased to 100 in the case of sausages made of Entericin CTC492 treated share and count decreased to 50 cfu/g after 8 days.

The pH evolution in control share as well as in Entericin CTC492 treated one was totally similar, for which it may be deduced that cfu/g recount decrease is due to the action of Entericin CTC492 and not to variations on meat dough acidity.

### INFORMATION ON MICROORGANISM DEPOSITE

A deposite of microorganism has been carried out, in accordance with Budapest Treaty's provisions on the international recognition of the deposit of microorganism for the purpose of Patent procedure, with the International Authority for Deposites CECT, Colección Española de Cultivos Tipo, de la Universidad de Valencia (Spanish Type Culture Colection, University of Valencia), Campus de Burjasot, 46100 Burjasot (Valencia), Spain.

| Applicant identification | CECT Number | Date of Deposite |
|---|---|---|
| Enterococcus faecium CTC492 | CECT 4453 | 07-14-93 |

The culture is deposited and is at public disposal without restrictions, under Budapest Treaty conditions, although said disposal cannot be interpreted as a licence for carrying out the object of the present invention, infringing present patent applicant rights.

SEQUENCE LISTING

SEQ. ID. NO.: 1

(i) SEQUENCE CHARACTERISTICS:
(A) LENGTH: 35
(B) TYPE: Amino acid
(C) STRANDEDNESS: Single
(D) TOPOLOGY: Unknown

(ii) MOLECULE TYPE: Protein

(vi) ORIGINAL SOURCE:
(A) ORGANISM: Enterococcus faecium CECT 4453
(B) CELL TYPE: Bacteria acido-láctea

(ix) FEATURE:
(A) NAME / KEY: Amino acid
(B) LOCATION: 0-35

(xi) SEQUENCE DESCRIPTION: SEQ. ID. NO.: 1

Thr Thr His Ser Gly Lys Tyr Tyr Gly Asn Gly Val Tyr Cys Thr Lys
1               5                   10                  15
Asn Lys Cys Thr Val Asp Thr Ala Lys Ala Thr Thr Xaa Ile Ala Gly
            20                  25                  30
Met Ser Ile
        35

## Claims

1. Bacteriocin against bacteria of the genus Listeria, characterized by being a peptide with a molecular weight of 4.282 Daltons which has the following amino-acid sequence:

Thr-Thr-His-Ser-Gly-Lys-Tyr-Tyr-Gly-Asn-Gly-Val-
Tyr-Cys-Thr-Lys-Asn-Lys-Cys-Thr-Val-Asp-Thr-Ala-Lys-Ala-
Thr-Thr-Xaa-Ile-Ala-Gly-Met-Ser-Ile.

2. Bacteriocin according to Claim 1, characterized by being obtained from a culture of the bacterial strain CTC492 of Enterococcus faecium, obtainable from CECT 4453.

3. Bacteriocin, according to Claim 1, characterized by being obtained by cloning an auxiliar strain of any suitable microorganism with a vector plasmid obtained from a suitable chromosomic fragment of the bacterial strain CTC492 of Enterococcus faecium, obtainable from CECT 4453.

4. Bacteriocin, according to any one of Claims from 1 to 3, characterized by being disposed as a watery suspension.

5. Bacteriocin, according to any one of Claims from 1 to 3, characterized by being disposed as a dried or lyophilized form.

6. Compositions of material characterized by containing the bacteriocin of Claim 1.

7. The bacterial strain CTC492 of Enterococcus faecium, obtainable from CECT 4453, capable of producing the bacteriocin of Claim 1.

8. Use of the bacteriocin of Claim 1 to prevent and avoid the growth and propagation of Listeria monocytogenes on foods.

9. Use of the bacteriocin of Claim 1, according to Claim 8, to prevent and avoid the growth and propagation of Listeria monocytogenes on meat and meat products.

10. Use of the bacteriocin of Claim 1, according to Claim 8, to prevent and avoid the growth and propagation of Listeria monocytogenes on milk and dairy products.
